# EUROPEAN PATENT APPLICATION

(11) **EP 4 124 385 A1**
(43) Date of publication of application: **01.02.2023**
(21) Application number: 21187797.2
(22) Date of filing: 26.07.2021
(51) Int. Cl.: B01L 3/00, B01F 33/45, B01L 7/00

(54) **ASSEMBLY AND METHOD FOR COMBINED NUCLEIC ACID PURIFICATION AND AMPLIFICATION**

(71) Applicant: Helaxy Inc., 768805 Singapore (SG)
(72) Inventor: NG, Yin Kum, 768805 Singapore (SG); TAN, Joo Poh, 510474 Singapore (SG); TEE, Su Yun, 640759 Singapore (SG)
(74) Representative: Danner, Stefan

(57) **Abstract**

The present invention relates to an assembly and a corresponding method for performing a combined assay of nucleic acid purification and amplification from a biological sample, in particular with regard to prepare a library for next generation sequencing. A unique rotary design of one or more fluidic cards in the assembly allows for moving of biological samples and reagents around the fluidic card(s) based on centrifugal force provided by rotary motion and coupled with an indexing mechanism. By orienting the fluidic card(s) appropriately the fluidic flow can be directed from and to the various chambers on the fluidic card(s) to perform the different assay steps without the need of batching during sample processing.

## Description

### TECHNICAL FIELD

The present invention relates to an assembly and a corresponding method for performing a combined assay of nucleic acid purification and amplification from a biological sample, in particular for the preparation of a library for next generation sequencing.

### BACKGROUND

Next generation sequencing (NGS) has evolved as a powerful tool for the high-throughput sequencing of nucleic acids, thus allowing for rapid progress in various applications such as genomic identification, genetic testing, drug discovery, and disease diagnosis (reviewed, e.g., in Goodwin, S. et al. (2016) Nat. Rev. Genet. 17, 333-351; Slatko, B.E. et al. (2018) Curr. Protoc. Mol. Biol. 122, e59; Hess, J.F. et al. (2020) Biotechnol. Adv. 41, 107537).

As NGS technology continues to advance, the volume of nucleic acids that can be sequenced at one time is increasing. This enables not only the processing of larger and more complex samples as well as an increase in the number of reads per sample, resulting in the detection of even very small sequence variations within that sample.

In the current NGS setting for diagnostics applications automation plays an important role in accelerating the processing of samples by reducing hands-on time as well as the risk of sample mix-up arising from the complex procedures required.

The NGS library preparation process is still largely manual requiring extensive experimental efforts and skilled operators with a turnaround time of up to several days. Large automated liquid handlers are in fact available to facilitate the preparation, but these instruments generally process samples in batches and require costly plastic consumables such as sample tips and plates. As a result, NGS analyses are generally performed in specialized laboratories.

In order for NGS technology becoming more widely available as routine test, the workflow needs to be performed in a simple and decentralized manner, for clinical settings with the possibility of near patient testing. In particular, instruments are required that allow for the preparation of NGS libraries without the requirement of sample batching, that is, instruments enabling *inter alia* a combined nucleic acid purification and amplification. Concomitantly, the methodology should be easy to handle and cost-effective

Hence, it is an object of the present invention to provide such instruments for combined nucleic acid purification and amplification, particularly in the context of next generation sequencing applications, as well as corresponding methods.

### SUMMARY OF THE INVENTION

In one aspect, the present invention relates to an assembly for performing a combined assay of nucleic acid purification and amplification from a biological sample, the assembly comprising: (i) one or more disc-shaped fluidic cards being arranged on a rotating platform; and (ii) a means for the indexing of the one or more fluidic cards on the rotating platform, the means comprising an indexing mechanism engaging with the rotating platform in order to generate intermittent rotary motion of the rotating platform; wherein the one or more disc-shaped fluidic cards comprises: (x.i) one or more receiving chambers for receiving the biological sample and one or more reagents for nucleic acid purification; (x.ii) a purification chamber for purifying the nucleic acid, the purification chamber having a concave bottom surface, being located in the center of the fluidic card, and being in fluid communication with the one or more receiving chambers, with a descending slope from the one or more receiving chambers to the purification chamber; (x.iii) one or more reaction chambers for amplifying the nucleic acid, the one or more reaction chambers having a concave bottom surface, being located at an outer part of the fluidic card on one side of the purification chamber, and each reaction chamber being in fluid communication with the purification chamber, with a descending slope from the reaction chambers to the purification chamber; and (x.iv) a waste chamber for collecting assay waste, the waste chamber comprising an absorbent material, being located at an outer part of the fluidic card substantially opposite to the one or more receiving chambers, and being in fluidic communication with each of the one or more reaction chambers and with the purification chamber.

In specific embodiments, the intermittent rotary motion generated by the indexing mechanism occurs in a predetermined angle, and in particular embodiments, the indexing mechanism is selected from the group consisting of a Geneva indexing mechanism and an electronically controlled stepper motor.

In preferred embodiments the one or more disc-shaped fluidic cards further comprises one or more of the following: (x.v) one or more receiving chambers for receiving one or more reagents for nucleic acid amplification, the one or more receiving chambers being in fluid communication with the one or more reaction chambers, with a descending slope from the one or more receiving chambers to the one or more reaction chambers; (x.vi) one or more elution chambers for diluting the purified nucleic acid, the one or more elution chambers being located adjacent to the purification chamber and being in fluid communication with the purification chamber and the one or more reaction chambers; (x.vii) one or more pooling and/or collecting chambers for pooling and collecting of the amplified nucleic acid produced in the one or more reaction chambers, the one or more pooling and/or collecting chambers being located at an outer part of the fluidic card adjacent to the one or more reaction chambers and being in fluidic communication with the one or more reaction chambers; (x.viii) one or more ventilation chambers being in fluid communication with the one or more reaction chambers and/or the waste chamber.

In specific embodiments, the one or more receiving chambers (x.i) comprise a sample receiving chamber and a reagent receiving chamber, and in particular embodiments, the fluid communication from the sample receiving chamber to the purification chamber merges with the fluid communication from the reagent receiving chamber to the purification chamber.

In further particular embodiments, (i) the fluid communication from the one or more receiving chambers (x.i) to the purification chamber is substantially perpendicular to the fluid communication from the purification chamber to the waste chamber; and/or (ii) the fluid communication from the one or more receiving chambers (x.i) to the purification chamber is substantially perpendicular to the fluid communication from the purification chamber to the one or more reaction chambers; and/or (iii) the fluid communication from the one or more pooling and/or collecting chambers to the one or more reaction chambers is substantially perpendicular to the fluid communication from the purification chamber to the one or more reaction chambers; and/or (iv) the fluid communication from the one or more reaction chambers to the purification chamber is substantially perpendicular to the fluid communication from the purification chamber to the waste chamber; and/or (v) in the presence of one or more elution chambers, the fluid communication from the one or more elution chambers to the one or more reaction chambers is substantially perpendicular to the fluid communication from the purification chamber to the one or more elution chambers, the fluid communication from the one or more receiving chambers (x.i) to the purification chamber is substantially perpendicular to the fluid communication from the purification chamber to the one or more elution chambers, and the fluid communication from the one or more pooling and/or collecting chambers to the one or more reaction chambers is substantially perpendicular to the fluid communication from the one or more elution chambers to the one or more reaction chambers.

In further particular embodiments, the one or more reaction chambers are provided with: (i) one or more heating and/or cooling elements allowing for the controlling of the temperature in the one or more reaction chambers; and/or (ii) means allowing for the sealing of the one or more reaction chambers and configured to allow for controlling fluid communication to and from the one or more reaction chambers, particularly wherein the means for sealing is selected from the group consisting of a revolving cap, a spherical cap, and an oil overlay.

In preferred embodiments, the assembly further comprises: (i) a mixing device being arranged below the purification chamber of the one or more fluidic cards, particularly wherein the mixing device is a magnetic mixing device; and/or (ii) one or more means for dispensing assay reagents to the one or more receiving chambers and/or the one or more reaction chambers, wherein the one or more means are arranged above the one or more fluidic cards, and particularly wherein the one or more means are one or more carousels for dispensing a plurality of reagents.

In further preferred embodiments, the assembly is part of an integrated platform for performing next generation sequencing.

In another aspect, the present invention relates to a method for performing a combined assay of nucleic acid purification and amplification from a biological sample, the method comprising: (i) providing an assembly as defined herein above; (ii) allowing the biological sample and the one or more reagents for nucleic acid purification provided to the one or more receiving chambers (x.i) to proceed to the purification chamber via the descending slope from the one or more receiving chambers to the purification chamber; (iii) purifying the nucleic acid from the biological sample in the purification chamber and immobilizing the purified nucleic acid; (iv) indexing of the one or more fluidic cards with the waste chamber being oriented radially outwards and applying rotary movement to the rotary platform, thus allowing the assay waste to proceed by centrifugal flow from the purification chamber to the waste chamber; (v) indexing of the one or more fluidic cards with the one or more reaction chambers being oriented radially outwards and applying rotary movement to the rotary platform, thus allowing the purified nucleic acid to proceed by centrifugal flow from the purification chamber to the one or more reaction chambers; or in the presence of the one or more elution chambers, indexing of the one or more fluidic cards with the one or more elution chambers being oriented radially outwards and applying rotary movement to the rotary platform, thus allowing the purified nucleic acid to proceed by centrifugal flow from the purification chamber to the one or more elution chambers, and after diluting the purified nucleic acid, indexing of the one or more fluidic cards with the one or more reaction chambers being oriented radially outwards and applying rotary movement to the rotary platform, thus allowing the purified nucleic acid to proceed by centrifugal flow from the one or more elution chambers to the one or more reaction chambers; (vi) amplifying the nucleic acid in the one or more reaction chambers, particularly by performing a polymerase-chain reaction; and (vii) indexing of the one or more fluidic cards with the one or more pooling and/or collection chambers being oriented radially outwards and applying rotary movement to the rotary platform, thus allowing the amplified nucleic acid to proceed by centrifugal flow from the one or more reaction chambers to the one or more pooling and/or collection chambers.

In specific embodiments, the biological sample is selected from the group consisting of blood, plasma, serum, saliva, urine, nasopharyngeal swab, oropharyngeal swab, and tissue specimens.

In preferred embodiments, the purification of the nucleic acid is performed by immobilizing the nucleic acid to a magnetic solid support, particularly to paramagnetic beads. Particularly preferably in this regard, the method further comprises one or more of the following: (i) releasing of the nucleic acid from the biological sample by providing a reagent for cell lysis to the one or more receiving chambers (x.i) and allowing it to proceed to the purification chamber; and/or (ii) one or more washing steps of the immobilized nucleic acid by providing a washing reagent to the one or more receiving chambers (x.i) and allowing it to proceed to the purification chamber; and/or (iii) eluting the immobilized nucleic acid from the magnetic solid support by providing an elution reagent to the one or more receiving chambers (x.i) and allowing it to proceed to the purification chamber.

In particular embodiments, the one or more reaction chambers are provided with reagents for nucleic acid amplification prior to performing step (vi), wherein the one or more reagents for nucleic acid amplification are provided to the one or more receiving chambers (x.v) and allowed to proceed to the one or more reaction chambers via the descending slope from the one or more receiving chambers to the one or more reaction chambers.

In further particular embodiments, the method further comprises: diluting of the amplified nucleic acid by providing a dilution reagent to the one or more receiving chambers (x.v) and allowing it to proceed to the one or more reaction chambers.

In preferred embodiments, the combined assay of nucleic acid purification and amplification is performed in a multiplex-format; and/or wherein the amplified nucleic acid represents a nucleic acid library for next generation sequencing.

### DESCRIPTION OF THE DRAWINGS

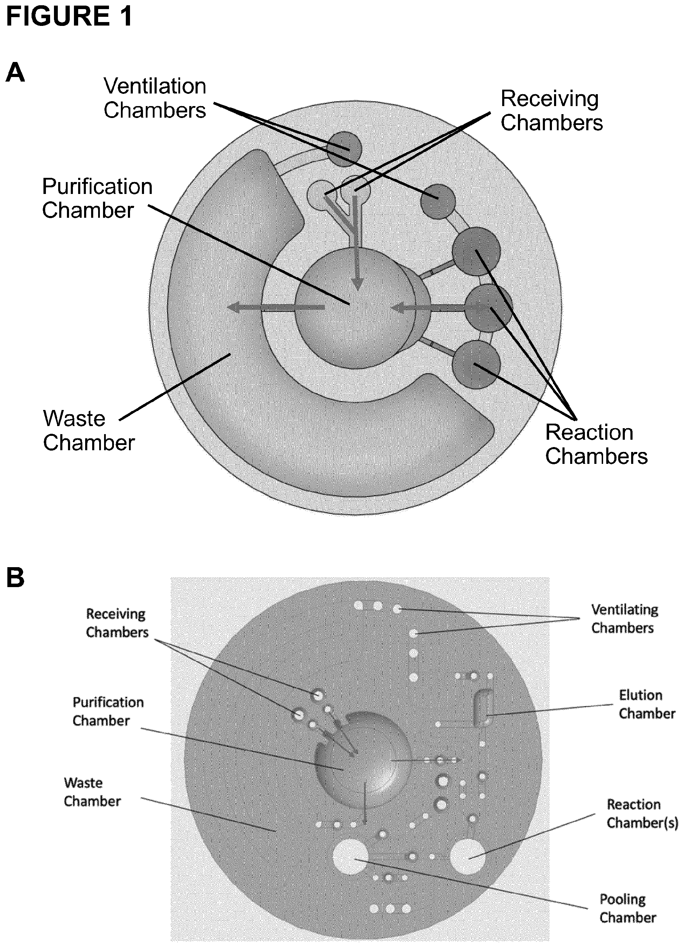

**FIGURE 1****: (A)** Schematic illustration of an exemplary fluidic card in accordance with the present invention. The fluidic card comprises two receiving chambers, for example, for introducing the biological sample and assay reagents, respectively, which are in fluidic communication via a channel with the central purification chamber for nucleic acid purification. The purification chamber, in turn, is in fluidic communication via respective channels with three reaction chambers for nucleic acid amplification as well as with a waste chamber for the disposal of assay waste. The reaction chambers and the waste chamber are each in fluidic communication with a ventilation chamber. Fluidic flow predominantly occurs from the receiving chambers to the purification chamber, from the purification chamber to the waste chamber, and between the purification chamber and the reaction chambers, respectively, as indicated by arrows. **(B)** Schematic illustration of another exemplary fluidic card in accordance with the present invention. The fluidic card comprises two receiving chambers in fluidic communication with the purification chamber. The purification chamber, in turn, is in fluidic communication with an elution chamber for diluting the purified nucleic acid before transfer to the one or more reaction chambers. The purification chamber is also in fluid communication with the waste chamber. The one or more reaction chambers are in fluid communication with the elution chamber and with a pooling chamber for pooling of the amplified nucleic acid. A plurality of ventilation chambers are in fluid communication with the other above-referenced chambers. Predominant fluidic flow is indicated by arrows.

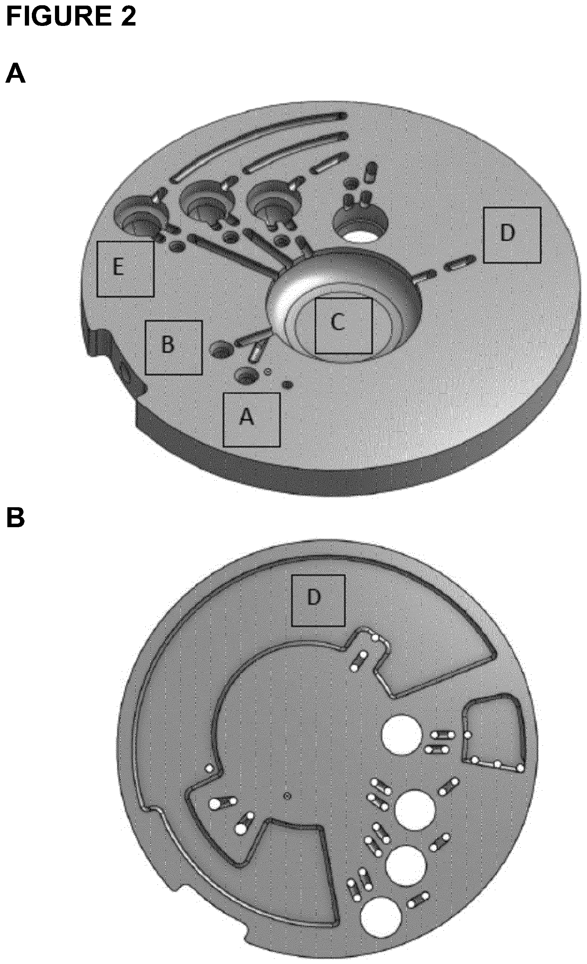

**FIGURE 2****:** Illustration of an exemplary fluidic card in accordance with the present invention. **(A)** top view; **(B)** bottom view. (A) and (B) represent receiving chambers for introducing the biological sample and assay reagents, respectively. (C) designates the purification chamber, (D) the waste chamber, and (E) three reaction chambers. In addition, various channels between chambers are indicated for accomplishing fluidic flow, as well as additional reagent ports in fluid communication with the reaction chambers for introducing reagents for nucleic acid amplification. The collection chamber (shown above the purification chamber) serves for pooling and collecting the amplified nucleic acid.

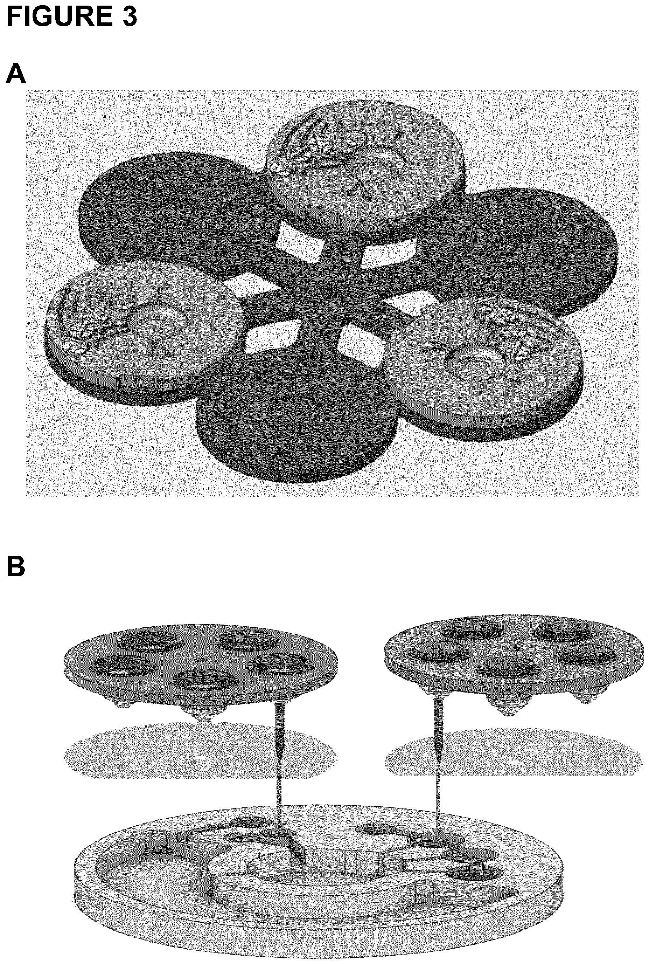

**FIGURE 3****: (A)** Illustration of an exemplary rotary platform in accordance with the present invention. Up to six fluidic cards can be arranged on the rotary platforms in order to process multiple biological samples simultaneously. Three fluidic cards as shown in Figure 2 are arranged on the rotary platform. In this embodiment, the reaction and collection chambers of the fluidic cards are each sealed by revolving caps. **(B)** Illustration of the exemplary fluidic card in combination with two reagent carousels for dispensing assay reagents to the receiving chambers for nucleic acid purification and the reaction chambers, respectively. For example, the receiving chambers are supplied with general reagents for nucleic acid purification (e.g., extraction and washing buffers), while the reaction chambers are supplied with specific reagents (e.g., primers) for nucleic acid amplification. Different reagents can be dispensed by rotating the reagent carousel itself. Multiple fluidic cards can be supplied by rotating each fluidic card to a particular position below the reagent carousel.

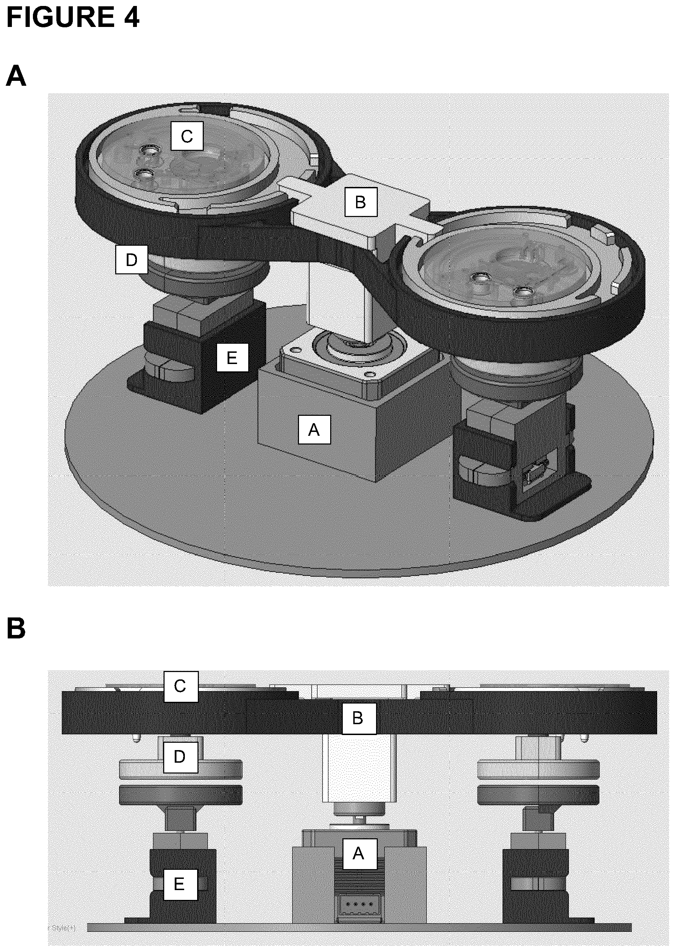

**FIGURE 4****:** Illustration of an exemplary assembly in accordance with the present invention. **(A)** perspective view; **(B)** side view. A rotary platform (B) on which two fluid discs (C) are arranged is mounted on a stepper motor (A) for rotating the platform, also employing an indexing mechanism to the rotary platform. Beneath each of the fluidic discs a magnetic mixing device (D) is arranged allowing for the mixing of the biological sample and the reagents. Each magnetic mixing device is in non-contact engagement with a motor (E).

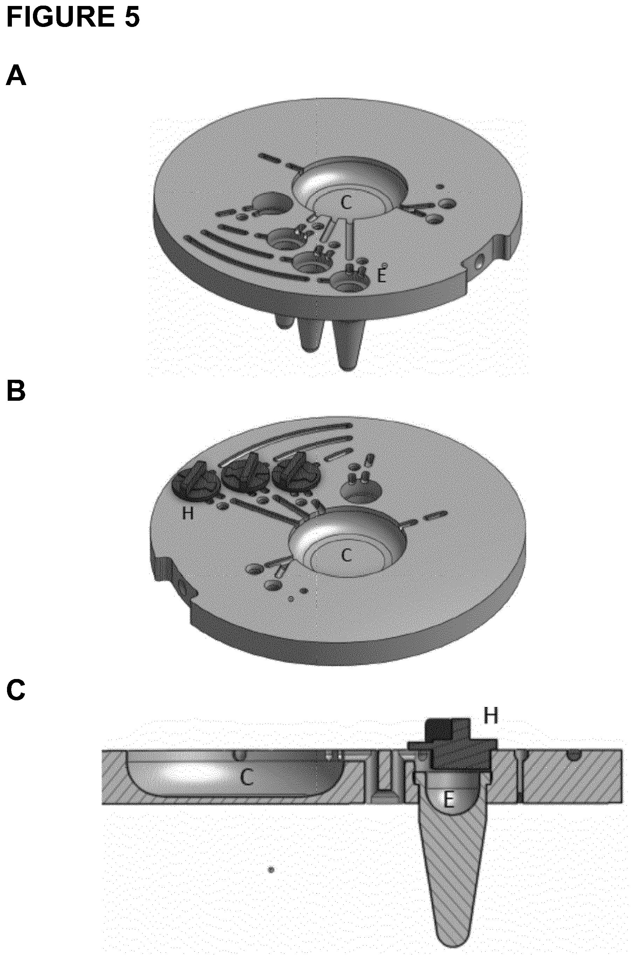

**FIGURE 5****:** Illustration of an exemplary fluidic card in accordance with the present invention as shown in Figure 2, wherein each of the reaction chambers (E) is provided with a heating and/or cooling element (shown as reaction vessel-like structure) allowing for the controlling of the temperature in the reaction chamber during nucleic acid amplification. Furthermore, each of the reaction chambers is equipped with a revolving cap (H) for the sealing of the reaction chamber and configured to allow for controlling fluid communication to and from the reaction chamber. **(A)** perspective view showing the heating and/or cooling elements; **(B)** perspective view showing the revolving caps; **(C)** cross-sectional view.

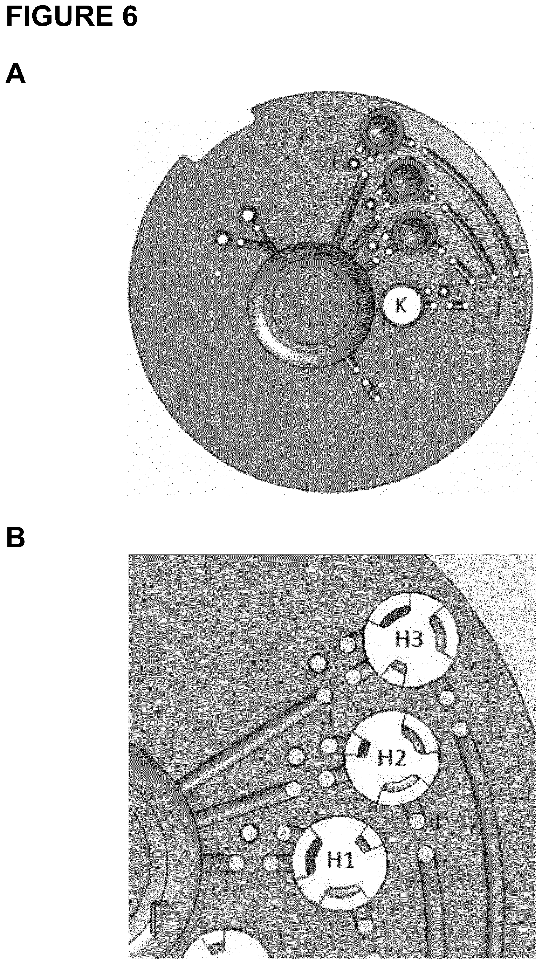

**FIGURE 6****: (A)** Illustration of an exemplary fluidic card in accordance with the present invention as shown in Figure 2, bottom view. (I) represent reagent ports in fluid communication with the respective reaction chambers for introducing reagents for nucleic acid amplification. (K) represents a pooling chamber for pooling the amplification products generated in the individual reaction chambers, and (K) represents a collection chamber for collection the final product. **(B)** Illustration of three different rotational positions of revolving caps (H) in their respective effects on the opening and closing of fluidic channels. Revolving cap H1 is in a position in which all three fluidic channels (to the purification chamber (C), the reagent port (I), and the pooling chamber (J), respectively) are open. Revolving cap H2 is in a position in which only the fluidic channels to the reagent port (I) and the pooling chamber (J) are open: Revolving cap H3 is in a position in which all three fluidic channels are closed.

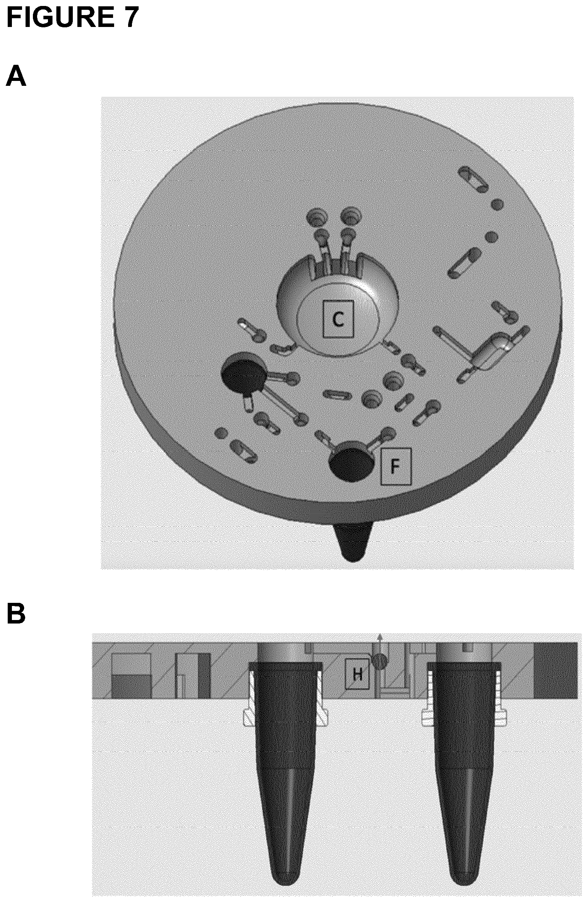

**FIGURE 7****:** Illustration of an exemplary fluidic card in accordance with the present invention as shown in Figure 1B. **(A)** perspective view; **(B)** cross-sectional view. Indicated are the purification chamber (C) and the reaction chamber (F). Fluid communication between the various chambers is controlled by "spherical caps", indicated as red spheres and as (H) in the cross-sectional view showing the fluid communication between purification/elution chamber and reaction chamber. The spherical caps, actually a sort of ball bearing, function as valves for unidirectional flow and to seal the one or more reaction chambers during nucleic acid amplification. Fluid communication between other chambers is controlled analogously.

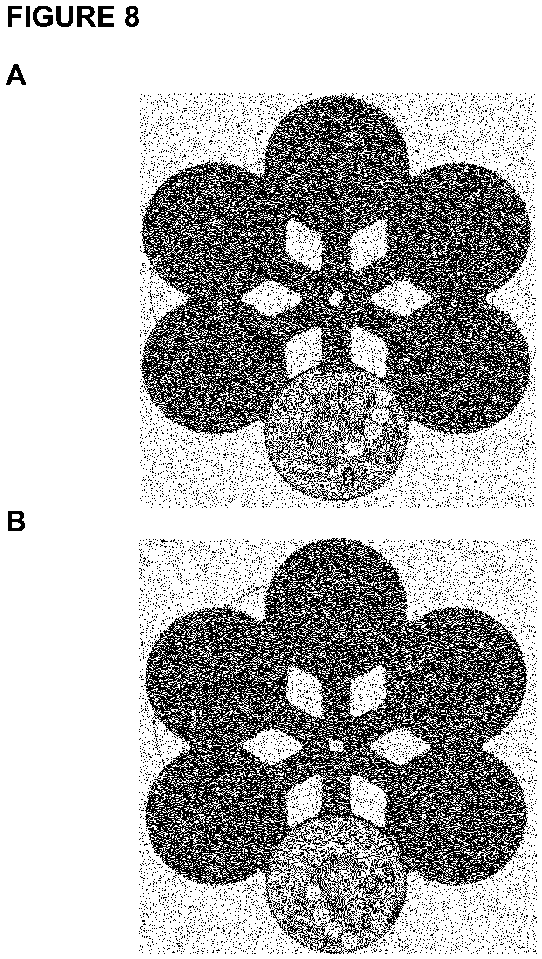

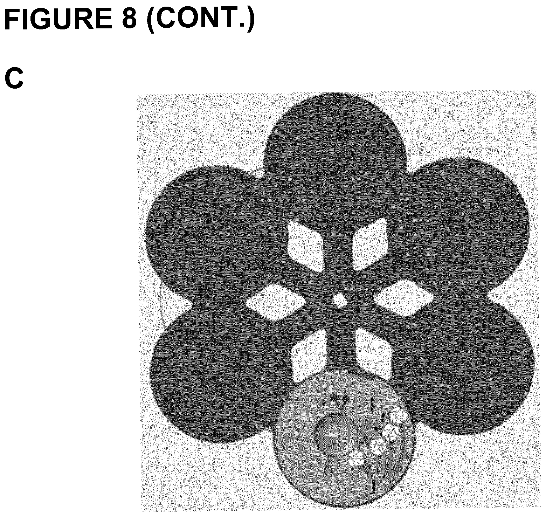

**FIGURE 8****:** Illustration of three exemplary steps of a method for performing a combined assay of nucleic acid purification and amplification in accordance with the present invention. The biological sample and the reagents for nucleic acid purification were introduced into the receiving chambers (A) and (B) of an exemplary fluidic card as shown in Figure 2, arranged on a rotary platform (G), and the nucleic acid was purified from the biological sample and immobilized in the purification chamber (C). **(A)** The fluidic card is indexed with the waste chamber being oriented radially outwards, and rotary movement is applied to the rotary platform (G), thus allowing the assay waste to proceed by centrifugal flow from the purification chamber to the waste chamber (D). Washing step scan be performed in a similar manner by adding washing buffer to receiving chamber (B), washing the immobilized nucleic acid in the purification chamber and discarding the waste in the waste chamber. **(B)** The immobilized nucleic acid is eluted by adding elution buffer to receiving chamber (B). The fluidic card is indexed with the reaction chambers (E) (which may be pre-filled with reagents for nucleic acid amplification) being oriented radially outwards, and rotary movement is applied to the rotary platform (G), thus allowing the purified nucleic acid to proceed by centrifugal flow from the purification chamber to the reaction chambers. **(C)** After nucleic acid amplification been completed the amplified product is diluted to the appropriate concentration. The fluidic card is indexed with the pooling chamber (J) and then the collection chamber being oriented radially outwards, and rotary movement is applied to the rotary platform (G), thus allowing the amplified nucleic acid to proceed by centrifugal flow from the reaction chambers to the pooling chamber and the collection chamber.

### DETAILED DESCRIPTION

The present invention is based on the unexpected finding that nucleic acids can be purified and amplified from a biological sample in a combined assay by means of particularly designed disc-shaped fluidic cards arranged on a rotary platform, with fluid movement around the fluidic cards be accomplished by centrifugal flow only, without a need for sophisticated syringes or fluidic pumps. Sample processing is coordinated by an indexing mechanism coupled to rotary movement. As multiple assays can be performed on the fluidic cards, no batching of samples is required. Assay reagents are integrated in the system and supplied via generic dispensing means by rotating the fluidic cards to the required position below the dispensing means. No plastic consumables are thus required for performing the assays. The methodology in accordance with the present invention provides for a significantly less laborious, simplified and cost-effective process, with a hands-on time of only minutes for loading the sample on the fluidic card and arranging the same on the rotary platform.

The process of the present invention is particularly applicable for the preparation of nucleic acid libraries for next generation sequencing. Library preparation can be completed within six hours, which is of high relevance *inter alia* in the field of diagnostics, where it may be essential to obtain results as fast as possible. Since no specialized laboratory is required for performing the assay, the present invention provides for a solution for near patent testing which further aides in improving timelines.

The present invention will be described in the following with respect to particular embodiments and with reference to certain drawings but is to be understood as not limited thereto but only by the appended claims. The present invention may suitably be practiced in the absence of any element(s) or limitation(s), not specifically disclosed herein.

Where the term "comprising" is used herein, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun, e.g., "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like, in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In one aspect, the present invention relates to an assembly for performing a combined assay of nucleic acid purification and amplification from a biological sample, the assembly comprising:
(i) one or more disc-shaped fluidic cards being arranged on a rotating platform; and
(ii) a means for the indexing of the one or more fluidic cards on the rotating platform, the means comprising an indexing mechanism engaging with the rotating platform in order to generate intermittent rotary motion of the rotating platform;
wherein the one or more disc-shaped fluidic cards comprises:
(x.i) one or more receiving chambers for receiving the biological sample and one or more reagents for nucleic acid purification;
(x.ii) a purification chamber for purifying the nucleic acid, the purification chamber having a concave bottom surface, being located in the center of the fluidic card, and being in fluid communication with the one or more receiving chambers, with a descending slope from the one or more receiving chambers to the purification chamber;
(x.iii) one or more reaction chambers for amplifying the nucleic acid, the one or more reaction chambers having a concave bottom surface, being located at an outer part of the fluidic card on one side of the purification chamber, and each reaction chamber being in fluid communication with the purification chamber, with a descending slope from the reaction chambers to the purification chamber; and
(x.iv) a waste chamber for collecting assay waste, the waste chamber comprising an absorbent material, being located at an outer part of the fluidic card substantially opposite to the one or more receiving chambers, and being in fluidic communication with each of the one or more reaction chambers and with the purification chamber.

The fluidic cards employed in the present invention have a disc-like shape, preferably a circular or nearly circular shape. Schematic illustrations of two exemplary fluidic cards employed herein are shown in Figure 1A and 1B, respectively. An exemplary design of such fluidic card is shown in Figure 2. The fluidic cards are arranged on a rotary platform. The rotary platform may hold a single fluidic card or may be configured to hold a plurality of fluidic cards, preferably an even number of fluidic cards, such as two, four, six, eight, ten or twelve fluidic cards. The one or more fluidic cards are arranged on the rotary platform in such manner that the fluidic cars can rotate themselves when the rotary platform is rotated. For example, the fluidic cards may be mounted on a holding means, such as a pin or plug at a respective position of the rotary platform, with which the fluidic cards are in rotary engagement. An exemplary rotary platform with fluidic cards arranged thereon is shown in Figure 3A.

The fluidic cards employed herein comprise various chambers which are described in detail below. Different chambers may be in fluid communication with each other, wherein fluid communication is preferably accomplished by channels between the chambers.

The fluidic card comprise one or more receiving chambers for receiving the biological sample and one or more reagents for nucleic acid purification. The one or more receiving chambers may be configured as ports or inlets to which the biological sample and the assay reagents are dispensed. In specific embodiments, the one or more receiving chambers comprise a separate sample receiving chamber and a separate reagent receiving chamber, which avoids the risk of contamination and enables the supply of different assay reagents for nucleic acid purification at certain points of time during sample processing (e.g., extraction reagents, washing buffers, and elution reagents).

The one or more receiving chambers are in fluid communication with the purification chamber where nucleic acid purification takes place. Fluid communication to the purification chamber may be separate for each of the one or more receiving chambers, for example, by means of separate channels that flow in the purification chamber. In other embodiments, the respective fluid communications from two or more receiving chambers merge prior to reaching the purification. In particular embodiments with separate sample and reagent receiving chambers, the fluid communication from the sample receiving chamber to the purification chamber merges with the fluid communication from the reagent receiving chamber to the purification chamber prior to reaching the purification chamber.

The fluid communication between the one or more receiving chambers and the purification chamber has a descending slope from the one or more receiving chambers to the purification chamber, thus allowing for a passive flow of biological sample and assay reagents to the purification chamber and also reducing the risk of any back-flow during sample processing in the purification chamber. To this purpose, in some embodiments, a step or drop is provided at the site where the fluid communication merges with the purification chamber so that there is another barrier for the back-flow of liquids. In addition, a "spherical cap" may be provided as a valve for ensuring unidirectional flow. Such spherical cap is a ball-like structure which functions like a ball bearing. The descending slope from the one or more receiving chambers to the purification chamber may be between 1% and 20%, such as 1%, 2%, 5%, 8%, 10%, 12%, 15%, 18%, and 20%.

The fluidic card also comprise a purification chamber for purifying the nucleic acid. The purification chamber is located in the center of the fluid card and has a concave bottom surface, that is, the bottom surfaced is curving in or hollowed inwards, towards the center. This configuration allows for containment of the liquid in the chamber and the processing of smaller liquid volumes. Typically, the purification chamber has a circular surface area or shape.

In preferred embodiments, the assembly further comprises a mixing device being arranged below the purification chamber of the one or more fluidic cards, particularly wherein the mixing device is a magnetic mixing device. The purification chamber may be equipped with an agitator or stirring device for mixing the liquid in the chamber. Preferably, the agitator or stirring device is magnetic, such as a magnetic stirring bar, that is driven by a magnetic mixer (e.g., a rotating magnet or an assembly of electromagnets). Alternatively, the agitator or stirring device may also be ferritic, such as one or more ferritic spheres, that is driven by a magnetic mixer. The agitator or stirring device may be mounted to the bottom of the purification chamber.

The fluidic card further provides one or more reaction chambers for amplifying the nucleic acid, the one or more reaction chambers having a concave bottom surface, that is, the bottom surfaced is curving in or hollowed inwards, towards the center. This configuration allows for containment and the processing of smaller liquid volumes. Typically, the fluid card provides for more than one rection chamber, such as two, three or four reaction chambers, which aids in facilitating nucleic acid amplification. Typically, the one or more reaction chambers have a circular surface area or shape. The one or more reaction chambers are located at an outer part of the fluidic card on one side of the purification chamber (and substantially opposite of the waste chamber, cf. below), and each reaction chamber is in fluid communication with the purification chamber. Fluid communication may be accomplished by separate channels from the purification chamber or by a common channel from the purification chamber which is diverging or branching off to establish a connection with each of the reaction chambers.

The fluid communication from the one or more reaction chambers to the purification chamber has a descending slope, thus allowing for a passive flow of the purified nucleic acid to the reaction chambers and also reducing the risk of any back-flow during nucleic acid processing (amplification) in the reaction chambers. To this purpose, in some embodiments, a step or drop is provided at the site where the fluid communication merges with the purification chamber so that there is another barrier for the back-flow of liquids. In addition, a "spherical cap" may be provided as a valve for ensuring unidirectional flow. The descending slope from the purification chamber to the one or more reaction chambers may be between 1% and 20%, such as 1%, 2%, 5%, 8%, 10%, 12%, 15%, 18%, and 20%.

In preferred embodiments, the fluidic card further comprises one or more elution chambers for diluting the purified nucleic acid prior to transfer to the one or more reaction chambers, thus ensuring that an appropriate amount of nucleic acid in an appropriate concentration is provided for nucleic acid amplification. The skilled person is well aware how as to determine the amount and/or concentration required for performing a particular nucleic acid amplification assay. The one or more elution chambers being located adjacent to the purification chamber and being in fluid communication with the purification chamber and the one or more reaction chambers. Typically, the fluid card provides for a single elution chamber.

The fluid communication from the one or more reaction chambers to the one or more elution chambers has a descending slope, thus allowing for a passive flow of the purified nucleic acid to the reaction chambers and also reducing the risk of any back-flow during nucleic acid processing (amplification) in the reaction chambers. To this purpose, in some embodiments, a step or drop is provided at the site where the fluid communication merges with the one or more elution chambers so that there is another barrier for the back-flow of liquids. In addition, a "spherical cap" may be provided as a valve for ensuring unidirectional flow. The descending slope from the one or more elution chambers to the one or more reaction chambers may be between 1% and 20%, such as 1%, 2%, 5%, 8%, 10%, 12%, 15%, 18%, and 20%.

In further preferred embodiments, the fluidic card further comprises one or more (additional) receiving chambers for receiving one or more reagents for nucleic acid amplification, the one or more receiving chambers being in fluid communication with the one or more reaction chambers, with a descending slope from the one or more receiving chambers to the one or more reaction chambers, thus allowing for a passive flow of the assay reagents to the reaction chambers and also reducing the risk of any back-flow during nucleic acid processing (amplification) in the reaction chambers. To this purpose, in some embodiments, a step or drop is provided at the site where the fluid communication merges with the one or more reaction chambers so that there is another barrier for the back-flow of liquids. In addition, a "spherical cap" may be provided as a valve for ensuring unidirectional flow. The descending slope from the one or more elution chambers to the one or more reaction chambers may be between 1% and 20%, such as 1%, 2%, 5%, 8%, 10%, 12%, 15%, 18%, and 20%.

The one or more receiving chambers may be configured as ports or inlets to which the assay reagents are dispensed. The descending slope of the fluid communication allows for a passive flow of the assay reagents to the reaction chambers and also reduces the risk of any back-flow of liquid. In some embodiments, one receiving chamber is provided for each reaction chamber, which would allow for performing different reactions in each reaction chamber by providing specific reagents (e.g., particular primers for nucleic acid amplification). In other embodiments, a common receiving chamber is available for providing reagents to several reaction chambers.

It would principally also be possible to introduce the reagents for nucleic acid amplification via the above-described one or more receiving chambers for one or more reagents for nucleic acid purifications, the reagents would then have to be transferred to the one or more reaction chambers indirectly via the purification chamber. However, such approach would require an additional transfer step.

In particular embodiments, the one or more reaction chambers are provided with one or more heating and/or cooling elements allowing for the controlling of the temperature in the one or more reaction chambers. Exemplary reaction chambers provided with heating and or cooling elements are shown in Figures 5A and 5C. Each of the one or more reaction chambers may be equipped with separate heating and/or cooling elements. Alternatively, a common heating and/or cooling element for all of the one or more reaction chambers may be provided. The heating and/or cooling elements may be configured as thermal block, well established as part of thermal cyclers used for performing polymerase chain reactions (PCR). Many of these thermal blocks make use of a Peltier element for thermoelectric heating and cooling, allowing for the raising and lowering of the temperature of the thermal block in discrete, predetermined steps, which is also known as thermocycling. The thermal block may have a gradient function, thus enabling different temperatures in different parts of the thermal block. In other embodiments, an inductive element is provided, which heats the reaction chambers via a non-contact method. In yet other embodiments, the heating and/or cooling elements are configured as a channel with hot and cold zones through which the reaction mixture is moved. Multiple elements for controlling the temperature during nucleic acid amplification (e.g., via PCR applications or isothermal amplification methods) are well established in the art and commercially available from different suppliers.

In further particular embodiments, the one or more reaction chambers are provided with means allowing for the sealing of the one or more reaction chambers to avoid evaporation during nucleic acid amplification. In some embodiments, sealing is accomplished by an overlay with oil, typically mineral oil. In other embodiments, the one or more reaction chambers are covered by a heated lid which also prevents the condensation of water from the reaction mixture inside the reaction chambers. In yet other embodiments, the one or more reaction chambers are provided with caps, preferably with revolving caps. Exemplary reaction chambers provided with revolving caps are shown in Figures 5B and 5C. Particularly, the means, such as the revolving caps, are configured to allow for controlling fluid communication to and from the one or more reaction chambers. In case of the revolving caps, this can be accomplished by openings or cavities in the cap which, when in a certain rotational position, enable fluid communication of the one or more reaction chambers with a first other chamber (e.g., the purification chamber or a receiving chamber for reagents for nucleic acid amplification). In another rotational position of the revolving cap, the fluid communication with the first other chamber is closed, but the fluid communication with a second other chamber is enabled. In yet another rotational position of the revolving cap, the fluid communication with both the first other chamber and the second other chamber is closed. In yet another rotational position of the revolving cap, the fluid communication with both the first other chamber and the second other chamber is enabled, and so on. Figure 6B illustrates an exemplary configuration of a revolving cap employed herein for controlling fluid communication to and from the one or more reaction chambers. Rotation of the revolving caps may be accomplished by means engaging with the revolving caps. The engagement may be coupled with the indexing mechanism engaging with the rotary platform of the assembly. In yet other embodiments, sealing of the one or more reaction chambers and control of fluid communication to and from the one or more reaction chambers are accomplished by ball bearing-like "spherical caps" positioned in the fluid communication, as described above. Exemplary reaction chambers being controlled by spherical caps are shown in Figure 7.

The fluidic card also comprises a waste chamber for collecting assay waste, both from nucleic acid purification and nucleic acid amplification. The waste chamber comprises an absorbent material (or a mixture of two or more different absorbent materials) for absorbing and binding of the liquid assay waste in order to avoid backflow from the waste chamber. Examples of suitable absorbent materials to be employed include cotton, cellulosic fibers, sponges as well as superabsorbent polymers, such as sodium polyacrylate, polyacrylamide copolymer, ethylene maleic anhydride copolymer, polyvinyl alcohol copolymer, cross-linked polyethylene oxide, cross-linked carboxymethylcellulose, and starch grafted copolymer of polyacrylonitrile, or combinations thereof. The absorbent material may be coated to one or more inner surfaces of the waste chamber, for example by means of an appropriate chemical agent., such as a cross-linking agent, or via UV cross-linking. The absorbent material (without liquid waste absorbed or bound) may cover at least 20%, at least 30%, at least 40%, at least 50%, at least 60%, or at least 70% of the volume of the waste chamber.

The waste chamber is located at an outer part of the fluidic card substantially opposite to the one or more receiving chambers. In this regard, the term "substantially opposite" is typically to be understood with regard to the respective fluid communications between (i) the one or receiving chambers and the purification chamber and (ii) the purification chamber and the waste chamber, which are substantially opposite to each other, that is, are typically arranged in an angle to each other in the range between 160° and 200°, preferably between 170° and 190° or between 175° and 185° or have a value of 180°. Alternatively, the arrangement is typically in an angle to each other in the range between 120° and 180°, preferably between 120° and 150° or between 150° and 180° or have a value of 135°. The waste chamber is the chamber with the largest size (in terms of volume and surface area) comprised on the fluid disc. The size of the waste chamber may cover at least 10%, at least 15%, at least 20%, at least 25%, at least 30%, at least 35%, at least 40%, at least 45% or at least 50% of the surface area of the fluidic card. With regard to the positioning of the waste chamber as such, it is located at an outer part of a large sector of the fluidic card, while the one or more reaction chamber are located substantially opposite on the remaining smaller sector of the outer part of the fluidic card (in certain embodiments along with the pooling chamber and/or the collection chamber, cf. below).

The waste chamber is in fluidic communication with each of the one or more reaction chambers and with the purification chamber. The waste chamber is in direct fluid communication with the purification chamber, typically accomplished via a channel, while the fluid communication of the waste chamber with the one or more reaction chambers occurs via the purification chamber. In some embodiments, the fluid communication between the waste chamber and the purification chamber has a descending slope from the waste chamber to the purification chamber, thus reducing the risk of any back-flow of waste during sample processing. To this purpose, in some embodiments, a step or drop is provided at the site where the fluid communication merges with the waste chamber so that there is another barrier for the back-flow of liquids. In addition, a "spherical cap" may be provided as a valve for ensuring unidirectional flow. The descending slope from the waste chamber to the purification chamber may be between 1% and 20%, such as 1%, 2%, 5%, 8%, 10%, 12%, 15%, 18%, and 20%.

In further preferred embodiments, the fluidic card further comprises one or more pooling and/or collecting chambers for pooling and/or collecting of the amplified nucleic acid produced in the one or more reaction chambers. The fluidic card may comprise a single chamber exerting both functions, the pooling and the collection of the nucleic acid. Alternatively, the fluidic card may comprise a separate pooling chamber and a separate collection chamber. In yet alternative embodiments, the fluidic card comprises one or more pooling chambers and one or more collection chambers. Typically, one pooling chamber and one collection chamber are provided.

The one or more pooling chambers for pooling the amplified nucleic acid produced in the one or more reaction chambers might be of particular relevance in case of different reactions performed in different reaction chamber (e.g., employing different amplification regimes or using different amplification primers), for example in order to prepare a nucleic acid library. Thus, the one or more pooling chambers are in fluid communication with the one or more reaction chambers. The one or more pooling chambers are located at an outer part of the fluidic card in proximity to the one or more reaction chambers.

The one or more collection chambers serve for collecting the amplified nucleic acid product and for removing it from the fluidic card. To the latter purpose, the one or more collection chambers have an opening for removing the amplified nucleic acid, for example, by means of a pipetting device. If both a pooling chamber and a collection chamber are provided, the collection chamber may be located between the central purification chamber and the pooling chamber. The arrangement of the pooling chamber, the collection chamber, and the one or more reaction chambers on the fluidic disc may be in form of a triangle, particularly in form of a right-angled triangle or a substantially right-angled triangle (with an angle in the range between 80° and 100°, and preferably between 85° and 95°). The collection chamber is in fluid communication with the pooling chamber, if the latter is present. If no separate pooling chamber is present, the collection chamber is in fluid communication with the one or more reaction chambers. Figure 6A illustrates an exemplary configuration of a fluidic card in accordance with the present invention comprising a pooling chamber and a collection chamber.

In particular embodiments, the fluid communication from the one or more receiving chambers (for the biological sample and the one or more assay reagents for nucleic acid purification) to the purification chamber is substantially perpendicular to the fluid communication from the purification chamber to the waste chamber. That is, in accordance with the present invention the respective fluid communications are typically arranged in an angle to each other in the range between 70° and 110°, preferably in the range between 80° and 100° or between 85° and 95°. or have a value of 90°. Alternatively, the arrangement is typically in an angle to each other in the range between 120° and 180°, preferably in the range between 120° and 150° or between 150° and 180°, or have a value of 135°.

In alternative particular embodiments, the fluid communication from the one or more receiving chambers (for the biological sample and the one or more assay reagents for nucleic acid purification) to the purification chamber is substantially opposite to the fluid communication from the purification chamber to the waste chamber, That is, in accordance with the present invention the respective fluid communications are typically arranged in an angle to each other in the range between 160° and 200°, preferably between 170° and 190° or between 175° and 185°, or have a value of 180°.

In further particular embodiments, the fluid communication from the one or more receiving chambers (for the biological sample and the one or more assay reagents for nucleic acid purification) to the purification chamber is substantially perpendicular to the fluid communication from the purification chamber to the one or more reaction chambers. That is, in accordance with the present invention the respective fluid communications are typically arranged in an angle to each other in the range between 70° and 110°, preferably in the range between 80° and 100° or between 85° and 95°, or have a value of 90°.

In yet further particular embodiments, the fluid communication from the one or more pooling and/or collecting chambers to the one or more reaction chambers is substantially perpendicular to the fluid communication from the purification chamber to the one or more reaction chambers.

More particularly, in case of a single collection chamber (optionally also exerting a function as pooling chamber), the fluid communication from the collecting chamber to the one or more reaction chambers is substantially perpendicular to the fluid communication from the purification chamber to the one or more reaction chambers. That is, in accordance with the present invention the respective fluid communications are typically arranged in an angle in the range between 70° and 110°, preferably in the range between 80° and 100° or between 85° and 95°, or have a value of 90°.

In alternative particular embodiments, in case of a separate pooling chamber and a separate collection chamber, the fluid communication from the pooling chamber to the one or more reaction chambers is substantially perpendicular to the fluid communication from the purification chamber to the one or more reaction chambers. That is, in accordance with the present invention the respective fluid communications are typically arranged in an angle in the range between 70° and 110°, preferably in the range between 80° and 100° or between 85° and 95°, or have a value of 90°. In further particular embodiments, the fluid communication from the pooling chamber to the one or more reaction chambers and the fluid communication from the pooling chamber to the collection chamber are typically arranged in an angle in the range between 25° and 65°, preferably in the range between 35° and 55° or between 40° and 50°, or have a value of 45°.

In yet further particular embodiments, the fluid communication from the one or more reaction chambers to the purification chamber is substantially perpendicular to the fluid communication from the purification chamber to the waste chamber. That is, the respective fluid communications are typically arranged in an angle to each other in the range between 70° and 110°, preferably in a range between 80° and 100° or between 85° and 95°, or have a value of 90°.

In yet further particular embodiments, in the presence of one or more elution chambers, the fluid communication from the one or more elution chambers to the one or more reaction chambers is substantially perpendicular to the fluid communication from the purification chamber to the one or more elution chambers, the fluid communication from the one or more receiving chambers (for the biological sample and the one or more assay reagents for nucleic acid purification) to the purification chamber is substantially perpendicular to the fluid communication from the purification chamber to the one or more elution chambers, and the fluid communication from the one or more pooling and/or collecting chambers to the one or more reaction chambers is substantially perpendicular to the fluid communication from the one or more elution chambers to the one or more reaction chambers. Thereby: (i) The fluid communication from one or more elution chambers to the one or more reaction chambers may be arranged with respect to the fluid communication from the purification chamber to the one or more elution chambers in an angle in the range between 70° and 110°, preferably in the range between 80° and 100° or between 85° and 95°, or have a value of 90°. (ii) The fluid communication from the one or more receiving chambers (for the biological sample and the one or more assay reagents for nucleic acid purification) to the purification chamber may be arranged with respect to the fluid communication from the purification chamber to one or more elution chambers in an angle in the range between 160° and 200°, preferably between 170° and 190° or between 175° and 185°, or have a value of 180°, or alternatively, in the range between 120° and 180°, preferably in the range between 120° and 150° or between 150° and 180°, or have a value of 135°. (iii) The fluid communication from the one or more pooling and/or collecting chambers to the one or more reaction chambers may be arranged with respect to the fluid communication from the one or more elution chambers to the one or more reaction chambers in an angle in the range between 70° and 110°, preferably in the range between 80° and 100° or between 85° and 95°, or have a value of 90°.

In further preferred embodiments, the fluidic card further comprises one or more ventilation chambers being in fluid communication with the one or more reaction chambers and/or the waste chamber and/or with the one or more elution chambers, if present. Typically, two ventilation chambers are provided, wherein a first ventilation chamber is in fluid communication with the one or more reaction chambers, and a second ventilation chamber is in fluid communication with the waste chamber. Alternatively, in the presence of one or more elution chambers, the two ventilation chambers are provided in fluid communication with the one or more elution chambers and in fluid communication with the waste chamber, respectively. The one or more ventilation chambers have opening for regulating airflow. In the simplest way such regulation of airflow can be the exhausting of air from the fluidic card. However, it is also possible to utilize pumps, preferably mini-pumps to regulate airflow. The pumps may be connected to the one or more ventilation chambers via tubes, pipes, or the like. A positive airflow can be generated by using pressured air, a negative airflow by using a vacuum, respectively. This can be achieved, for example, by alternating the direct current supply polarity to the pumps. Regulation of the airflow within the fluidic card to direct or facilitate liquid flow through internal fluid communications (e.g., channels) or to facilitate the removal of any vapors.

In further preferred embodiments, the assembly of the present invention further comprises one or more means for dispensing assay reagents to the one or more receiving chambers and/or the one or more reaction chambers, wherein the term "receiving chamber" comprises both the one or more receiving chambers for (the biological sample and) the reagents for nucleic acid purification as well as the one or more (additional) receiving chambers for the reagents for nucleic acid amplification. The one or more dispensing means are arranged in the assembly above the one or more fluidic cards. Typically, such dispensing means comprises a container for the reagent supply and a tip or nozzle through which the reagent is dispensed. Preferably, automated or programmable dispensing means are provided which dispense a predetermined amount of a given reagent to a certain chamber when the dispensing means as reached a predetermined position in proximity to said chamber. Various automated or programmable dispensing means are established in the art and commercially available from different suppliers.

The dispensing means may be configured to dispense a single reagent or, preferably, the dispensing means comprises a plurality of containers for the dispensing of different reagent. In particularly preferred embodiments, the dispensing means are carousels for the dispensing of different reagents, which can be rotated such that a certain reagent can be specifically dispensed to a certain chamber in the fluidic card. The one or more dispensing means are arranged above the one or more fluidic cards so that their tips or nozzles can be readily and accurately positioned and the reagents introduced in the respective chambers from the top. Such arrangement allows for using the same supply of one or more reagents for the concomitant processing of different samples on different fluidic cards or for the serial processing of different samples on one fluidic card or for different assays to be performed on the one or more fluidic cards. Different dispensing means, such as carousels, may be employed for the supply with general reagents (such as washing buffers or buffers for diluting the sample) and for the supply with specific reagents (such as amplification primers), respectively. The positioning of the dispensing means (e.g., the rotational position of a reagent carousel) may be determined and regulated by an indexing mechanism for generating an intermittent rotary motion. The indexing mechanism for the positioning of the dispensing means may be the same or be coupled to the indexing mechanism engaging with the rotating platform in order to generate intermittent rotary motion of the rotating platform cf. below). Figure 3B shows an exemplary fluidic card combination with two reagent carousels for dispensing assay reagents to the receiving chambers and the reaction chambers, respectively.

The assembly in accordance with the present invention comprises a means for the indexing of the one or more fluidic cards on the rotating platform, the means comprising an indexing mechanism engaging with the rotating platform in order to generate intermittent rotary motion of the rotating platform. In other words, the rotating platform does not rotate continuously, but intermittently with alternate periods of motion and rest, optionally with no reversal in direction. In preferred embodiments, the intermittent rotary motion generated by the indexing mechanism occurs in a predetermined angle in order to allow the one or more fluidic cards to rotate to the desired position for performing a particular method step. Typically, the rotational angle is predetermined in a regular manner, that is, the angle is always the same. For example, the angle may be 180° so that the rotating platform has two stops per full rotation (at 180° and 360°), or the angle may be 90° so that the rotating platform has four stops per full rotation (at 90°, 180°, 270°, and 360°), or the angle may be 60° so that the rotating platform has six stops per full rotation (at 60°, 120°, 180° 240°, 300°, and 360°), and so on. It is principally also possible that different predetermined angles are used.

The indexing mechanism engages with the rotating platform in order to generate intermittent rotary motion. In a preferred embodiment, the indexing mechanism is a Geneva indexing mechanism (also known as "Maltese cross") well established in the art. Here, a rotating drive wheel (also known as cam wheel) is usually equipped with a pin on the top surface that engages in a slot located in another wheel (i.e., the driven wheel or follower wheel that is arranged beneath the rotating platform) having a regular pattern of such slots. The engagement of the pin in the driven wheel results in an intermittent rotary movement of the driven wheel for a certain angle, particularly a predetermined angle. The drive wheel also has a circular blocking disc arranged on the top surface of the drive wheel and having a recess opposite of the pin. The blocking disc holds the driven wheel in position until the pin engages with the next slot. In an alternative preferred embodiment, the indexing mechanism is an electronically controlled stepper motor which is typically a brushless direct currency motor that divides a full rotation into a number of equal steps. Stepper motors are well known in the art. Stepper motors have multiple "toothed" electromagnets arranged as a stator around a central rotor, typically a gear-shaped iron wheel. The electromagnets are energized by an external driver circuit or micro-controlle. In order to rotate the motor shaft one electromagnet is given power, which magnetically attracts the gear's teeth resulting in an engagement. When the gear's teeth are aligned to the first electromagnet, they are slightly offset from the next electromagnet. Thus, when the next electromagnet is given power and power is removed from the first one, the gear rotates slightly to align with the next one. In that way, the motor can be turned by a precise angle.

Figure 4 illustrates an exemplary assembly in accordance with the present invention. A rotary platform (B) on which two fluid discs (C) are arranged is mounted on a stepper motor (A) for rotating the platform, also employing an indexing mechanism to the rotary platform. Beneath each of the fluidic discs a magnetic mixing device (D) is arranged allowing for the mixing of the biological sample and the reagents. Each magnetic mixing device is in non-contact engagement with a stepper motor (E). The respective predetermined angles by which the stepper motor (A) intermittently rotates the rotary platform (B) and the stepper motor (E) intermittently rotates the mixing device (D) are synchronized or aligned to each other in order to enable accurate and efficient sample processing.

In a preferred embodiment, the assembly described herein above is used for the preparation of a nucleic acid library, in particular a library for nucleic acid sequencing.

In another preferred embodiment, the assembly is part of an integrated platform for performing next generation sequencing, that is, the assembly for nucleic acid purification and amplification is combined with a sequencing

In another aspect, the present invention relates to a method for performing a combined assay of nucleic acid purification and amplification from a biological sample, the method comprising:
(i) providing an assembly as defined herein above;
(ii) allowing the biological sample and the one or more reagents for nucleic acid purification provided to the one or more receiving chambers (x.i) to proceed to the purification chamber via the descending slope from the one or more receiving chambers to the purification chamber;
(iii) purifying the nucleic acid from the biological sample in the purification chamber and immobilizing the purified nucleic acid;
(iv) indexing of the one or more fluidic cards with the waste chamber being oriented radially outwards and applying rotary movement to the rotary platform, thus allowing the assay waste to proceed by centrifugal flow from the purification chamber to the waste chamber;
(v) indexing of the one or more fluidic cards with the one or more reaction chambers being oriented radially outwards and applying rotary movement to the rotary platform, thus allowing the purified nucleic acid to proceed by centrifugal flow from the purification chamber to the one or more reaction chambers; or
   in the presence of the one or more elution chambers, indexing of the one or more fluidic cards with the one or more elution chambers being oriented radially outwards and applying rotary movement to the rotary platform, thus allowing the purified nucleic acid to proceed by centrifugal flow from the purification chamber to the one or more elution chambers, and after diluting the purified nucleic acid, indexing of the one or more fluidic cards with the one or more reaction chambers being oriented radially outwards and applying rotary movement to the rotary platform, thus allowing the purified nucleic acid to proceed by centrifugal flow from the one or more elution chambers to the one or more reaction chambers;
(vi) amplifying the nucleic acid in the one or more reaction chambers, particularly by performing a polymerase-chain reaction; and
(vii) indexing of the one or more fluidic cards with the one or more pooling and/or collection chambers being oriented radially outwards and applying rotary movement to the rotary platform, thus allowing the amplified nucleic acid to proceed by centrifugal flow from the one or more reaction chambers to the one or more pooling and/or collection chambers.

The respective protocols employed in connection with the present invention for nucleic acid purification and for nucleic acid amplification follow established standard procedures known in the art (cf., for example, Sambrook, J. et al. (1989) Molecular Cloning: A Laboratory Manual. 2nd Ed., Cold Spring Harbor Laboratory Press, Cold Spring Harbor, NY; Ausubel, F.M. et al. (2001) Current Protocols in Molecular Biology. Wiley & Sons, Hoboken, NJ).

The biological sample analyzed herein is sample derived from a mammal such as a mouse, rat, hamster, rabbit, cat, dog, pig, cow, horse or monkey, and preferably a human. Such samples may include body tissues (e.g., biopsies or resections, tissue specimens, such as formalin-fixed, paraffin-embedded tissue), swabs (e.g., nasopharyngeal or oropharyngeal swabs, wound swabs), stool samples, and body fluids, such as blood (whole blood, plasma, serum), saliva, sputum, urine, and cerebrospinal fluid. The samples may contain a single cell, a cell population (i.e., two or more cells) or a cell extract or a suspension derived from a body tissue or swab, and may be used in unpurified form or subjected to any enrichment or purification step(s) prior to use. The skilled person is well aware of various such purification methods (see, e.g., Sambrook, J., and Russel, D.W. (2001), *supra;* Ausubel, F.M. et al. (2001) *supra*). The term "whole blood" refers to blood with all its constituents (i.e., both blood cells and plasma). The term "plasma" denotes the blood's liquid medium. The term "serum" refers to plasma from which the clotting proteins have been removed. In particular embodiments, the biological sample is selected from the group consisting of blood, plasma, serum, saliva, urine, nasopharyngeal swab, oropharyngeal swab, and tissue specimens.

The sample is introduced in the one or more respective receiving chambers for the biological sample and the one or more reagents for nucleic acid purification of a fluidic card in accordance with the present invention and allowing it to procced to the purification chamber via the descending slope from the one or more receiving chambers to the purification chamber. Preferably, the biological sample is introduced to a specific receiving chamber for the biological sample only in order to reduce the risk of any contaminations. The one or more reagents for nucleic acid purification may be introduced via the one or more dispensing means to the same receiving chamber as the biological sample and allowed to proceed to the purification chamber via the descending slope from the one or more receiving chambers to the purification chamber Preferably, the one or more reagents for nucleic acid purification are introduced to a separate receiving chamber. The reagents may be introduced to the fluidic card concomitantly with the biological sample or after the sample been introduced. It may also be possible to introduce some of reagents to the respective receiving chamber (for example, reagents for cell lysis) before introducing the biological sample. Typically, no centrifugal flow is required for allowing biological sample and assay reagents to proceed from the one or more receiving chambers to the purification chamber.

The nucleic acid is then purified in the purification chamber and immobilized to a support, preferably a solid support. The skilled person is well aware of selecting an appropriate protocol for performing the purification assay. For example, the protocol to be employed will *inter alia* depend on the type of nucleic acid that needs to be purified, ribonucleic acid (RNA) or deoxyribonucleic acid (DNA), the length of the nucleic acid (e.g., genomic DNA or coding DNA, mRNA or small RNAs, such as microRNAs), and the like. The nucleic acids may be naturally occurring or recombinantly produced.

Preferably, the nucleic acid purification comprises and extraction or cell lysis step (e.g., using an alkaline lysis buffer or certain enzymes, such as alkaline protease) to break the cell walls and release the nucleic acid from the cells. After immobilization of the nucleic acid, one or more washing steps and performed to remove cell debris and other contaminants. Finally, the purified nucleic acid is eluted from the support (see, e.g., Sambrook, J., and Russel, D.W. (2001), *supra;* Ausubel, F.M. et al. (2001) *supra*). For this purpose, in particular embodiments, the method further comprises (i) releasing of the nucleic acid from the biological sample by providing a reagent for cell lysis to the one or more receiving chambers and allowing it to proceed to the purification chamber; and/or (ii) one or more washing steps of the immobilized nucleic acid by providing a washing reagent to the one or more receiving chambers and allowing it to proceed to the purification chamber; and/or (iii) eluting the immobilized nucleic acid from the magnetic solid support by providing an elution reagent to the one or more receiving chambers and allowing it to proceed to the purification chamber.

After the addition of each reagent for nucleic acid purification the reaction mixture in the purification chamber may be mixed with the mixing device, preferably a magnetic mixing device, being arranged below the purification chamber.

Furthermore, after completion of each of these reaction steps (as well as after each of the washing steps) the assay waste is removed from the purification chamber by indexing of the one or more fluidic cards with the waste chamber (and particularly the fluid communication from the purification chamber to the waste chamber) being oriented radially outwards and applying rotary movement to the rotary platform, thus allowing the assay waste to proceed by centrifugal flow from the purification chamber to the waste chamber. This step is illustrated in Figure 8A.

In a further preferred embodiment, the purification of the nucleic acid is performed by immobilizing the nucleic acid to a magnetic solid support, particularly to paramagnetic beads. Magnetic separation technology is well established for the purification of nucleic acids. Paramagnetic beads are commercially available from various suppliers. Paramagnetic beads have a functionalized surface in order to facilitate nucleic acid binding. For example, the beads may be functionalized with a carboxyl-modified polymer or with silica. In case, the nucleic acid to be purified is mRNA, the paramagnetic beads may also be functionalized with oligo-dT. Immobilization of the nucleic acid bound to paramagnetic beads may be accomplished by the magnetic mixing device preferably arranged below the purification chamber.

Subsequently, the purified nucleic acid is transferred to the one or more reaction chambers by indexing of the one or more fluidic cards with the one or more reaction chambers (and particularly the fluid communication from the purification chamber to the one or more reaction chambers) being oriented radially outwards and applying rotary movement to the rotary platform, thus allowing the purified nucleic acid to proceed by centrifugal flow from the purification chamber to the one or more reaction chamber. This step is illustrated in Figure 8B.

Alternatively, in the presence of the one or more elution chambers, the purified nucleic acid is transferred to the one or more elution chambers by indexing of the one or more fluidic cards with the one or more elution chambers being oriented radially outwards and applying rotary movement to the rotary platform, thus allowing the purified nucleic acid to proceed by centrifugal flow from the purification chamber to the one or more elution chambers. After diluting the purified nucleic acid, the purified nucleic acid is transferred to the one or more reaction chambers by indexing of the one or more fluidic cards with the one or more reaction chambers being oriented radially outwards and applying rotary movement to the rotary platform, thus allowing the purified nucleic acid to proceed by centrifugal flow from the one or more elution chambers to the one or more reaction chambers.

In the one or more reaction chambers, the nucleic acid is amplified. Particularly, nucleic acid amplification is performed by polymerase chain reaction (PCR). However, other amplification protocols, such as isothermal amplification, are also possible. Nucleic acid amplification methods are well established in the art (see, e.g., Sambrook, J., and Russel, D.W. (2001), *supra;* Ausubel, F.M. et al. (2001) *supra*). Heating and/or cooling systems are employed for regulating the temperature in the one or more reaction chambers.

The same amplification reaction may be performed in all of the one or more reaction chambers, that is, the same reagents (such as amplification primers) and the same temperature profile (isothermal or thermocycling) are used. Alternatively, different reactions may be performed in different reaction chambers. In case of RNA being purified from the biological sample, the RNA may be reverse transcribed into cDNA by means of reverse transcriptase (RT) prior to amplification. This reaction may also be performed via a PCR application, RT-PCR.

The reagents required for performing the nucleic acid amplification reaction may be introduced via the one or more dispensing means directly in the one or more reaction chambers. Preferably, the reagents are introduced into the (additional) one or more receiving chambers in fluid communication with the one or more reaction chambers and allowed to procced to the purification chamber via the descending slope from the one or more receiving chambers to the one or more reaction chambers. Generic reagents (e.g., deoxyribonucleotides) may be supplied from one dispensing means, such as a reagent carousel, while specific reagents (e.g., primers) may be supplied from another dispensing means. The reagents may be introduced to the fluidic card after transfer of the purified nucleic acid from the purification chamber to the one or more reaction chambers. In particular embodiments, some of reagents for nucleic acid amplification to the one or more (additional) receiving chamber prior to the transfer of the nucleic acid and start of the amplification reaction. Typically, no centrifugal flow is required for allowing the assay reagents to proceed from the one or more receiving chambers to the one or more reaction chambers.

In particular embodiments, after completion of the amplification reaction the amplified nucleic acid is diluted to an appropriate concentration for further processing by adding water or a suitable other buffer directly to the one or more reaction chambers or, preferably, to the one or more (additional) receiving chambers.

In case, the one or more fluidic cards comprise (one or more) pooling chamber(s), the one or more fluidic cards are indexed with the pooling chamber (and particularly the fluid communication from the one or more reaction chambers to the pooling chamber) being oriented radially outwards, and rotary movement is applied to the rotary platform, thus allowing the amplified nucleic acid to proceed by centrifugal flow from the one or more reaction chamber to the pooling chamber. Subsequently, the amplified nucleic acid is transferred to the (one or more) collection chamber(s) by indexing of the one or more fluidic cards with the collection chamber (and particularly the fluid communication from the pooling chamber to the collection chamber) being oriented radially outwards and applying rotary movement to the rotary platform, thus allowing the amplified nucleic acid to proceed by centrifugal flow from the pooling chamber to the collection chamber. The amplified nucleic acid is then collected, removed from the one or more fluidic cards, and subjected to further processing, if applicable. This step is illustrated in Figure 8C.

Alternatively, in the absence of a particular pooling chamber, the one or more fluidic cards are indexed with the (one or more) collection chamber(s) (and particularly the fluid communication from the one or more reaction chambers to the collection chamber) being oriented radially outwards and applying rotary movement to the rotary platform, thus allowing the amplified nucleic acid to proceed by centrifugal flow from the one or more reaction chambers to the collection chamber. The amplified nucleic acid is then collected, removed from the one or more fluidic cards, and subjected to further processing, if applicable

In preferred embodiments, the combined assay of nucleic acid purification and amplification is performed in a multiplex-format.

In further preferred embodiments, the amplified nucleic acid represents a nucleic acid library, and particularly a nucleic acid library for next generation sequencing.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. An assembly for performing a combined assay of nucleic acid purification and amplification from a biological sample, the assembly comprising:
(i) one or more disc-shaped fluidic cards being arranged on a rotating platform; and
(ii) a means for the indexing of the one or more fluidic cards on the rotating platform, the means comprising an indexing mechanism engaging with the rotating platform in order to generate intermittent rotary motion of the rotating platform;
wherein the one or more disc-shaped fluidic cards comprises:
(x.i) one or more receiving chambers for receiving the biological sample and one or more reagents for nucleic acid purification;
(x.ii) a purification chamber for purifying the nucleic acid, the purification chamber having a concave bottom surface, being located in the center of the fluidic card, and being in fluid communication with the one or more receiving chambers, with a descending slope from the one or more receiving chambers to the purification chamber;
(x.iii) one or more reaction chambers for amplifying the nucleic acid, the one or more reaction chambers having a concave bottom surface, being located at an outer part of the fluidic card on one side of the purification chamber, and each reaction chamber being in fluid communication with the purification chamber, with a descending slope from the reaction chambers to the purification chamber; and
(x.iv) a waste chamber for collecting assay waste, the waste chamber comprising an absorbent material, being located at an outer part of the fluidic card substantially opposite to the one or more receiving chambers, and being in fluidic communication with each of the one or more reaction chambers and with the purification chamber.

2. The assembly of claim 1, wherein the intermittent rotary motion generated by the indexing mechanism occurs in a predetermined angle, and particularly wherein the indexing mechanism is selected from the group consisting of a Geneva indexing mechanism and an electronically controlled stepper motor.

3. The assembly of claim 1 or 2, wherein the one or more disc-shaped fluidic cards further comprises one or more of the following:
(x.v) one or more receiving chambers for receiving one or more reagents for nucleic acid amplification, the one or more receiving chambers being in fluid communication with the one or more reaction chambers, with a descending slope from the one or more receiving chambers to the one or more reaction chambers;
(x.vi) one or more elution chambers for diluting the purified nucleic acid, the one or more elution chambers being located adjacent to the purification chamber and being in fluid communication with the purification chamber and the one or more reaction chambers;
(x.vii) one or more pooling and/or collecting chambers for pooling and collecting of the amplified nucleic acid produced in the one or more reaction chambers, the one or more pooling and/or collecting chambers being located at an outer part of the fluidic card adjacent to the one or more reaction chambers and being in fluidic communication with the one or more reaction chambers;
(x.viii) one or more ventilation chambers being in fluid communication with the one or more reaction chambers and/or the waste chamber.

4. The assembly of any one of claims 1 to 3, wherein the one or more receiving chambers (x.i) comprise a sample receiving chamber and a reagent receiving chamber, and particularly wherein the fluid communication from the sample receiving chamber to the purification chamber merges with the fluid communication from the reagent receiving chamber to the purification chamber.

5. The assembly of any one of claims 1 to 4, wherein:
(i) the fluid communication from the one or more receiving chambers (x.i) to the purification chamber is substantially perpendicular to the fluid communication from the purification chamber to the waste chamber; and/or
(ii) the fluid communication from the one or more receiving chambers (x.i) to the purification chamber is substantially perpendicular to the fluid communication from the purification chamber to the one or more reaction chambers; and/or
(iii) the fluid communication from the one or more pooling and/or collecting chambers to the one or more reaction chambers is substantially perpendicular to the fluid communication from the purification chamber to the one or more reaction chambers; and/or
(iv) the fluid communication from the one or more reaction chambers to the purification chamber is substantially perpendicular to the fluid communication from the purification chamber to the waste chamber; and/or
(v) in the presence of one or more elution chambers, the fluid communication from the one or more elution chambers to the one or more reaction chambers is substantially perpendicular to the fluid communication from the purification chamber to the one or more elution chambers, the fluid communication from the one or more receiving chambers (x.i) to the purification chamber is substantially perpendicular to the fluid communication from the purification chamber to the one or more elution chambers, and the fluid communication from the one or more pooling and/or collecting chambers to the one or more reaction chambers is substantially perpendicular to the fluid communication from the one or more elution chambers to the one or more reaction chambers.

6. The assembly of any one of claims 1 to 5, wherein the one or more reaction chambers are provided with:
(i) one or more heating and/or cooling elements allowing for the controlling of the temperature in the one or more reaction chambers; and/or
(ii) means allowing for the sealing of the one or more reaction chambers and configured to allow for controlling fluid communication to and from the one or more reaction chambers, particularly wherein the means for sealing is selected from the group consisting of a revolving cap, a spherical cap, and an oil overlay.

7. The assembly of any one of claims 1 to 6, further comprising:
(i) a mixing device being arranged below the purification chamber of the one or more fluidic cards, particularly wherein the mixing device is a magnetic mixing device; and/or
(ii) one or more means for dispensing assay reagents to the one or more receiving chambers and/or the one or more reaction chambers, wherein the one or more means are arranged above the one or more fluidic cards, and particularly wherein the one or more means are one or more carousels for dispensing a plurality of reagents.

8. The assembly of any one of claims 1 to 7, being part of an integrated platform for performing next generation sequencing.

9. A method for performing a combined assay of nucleic acid purification and amplification from a biological sample, the method comprising:
(i) providing an assembly as defined in any one of claims 1 to 8;
(ii) allowing the biological sample and the one or more reagents for nucleic acid purification provided to the one or more receiving chambers (x.i) to proceed to the purification chamber via the descending slope from the one or more receiving chambers to the purification chamber;
(iii) purifying the nucleic acid from the biological sample in the purification chamber and immobilizing the purified nucleic acid;
(iv) indexing of the one or more fluidic cards with the waste chamber being oriented radially outwards and applying rotary movement to the rotary platform, thus allowing the assay waste to proceed by centrifugal flow from the purification chamber to the waste chamber;
(v) indexing of the one or more fluidic cards with the one or more reaction chambers being oriented radially outwards and applying rotary movement to the rotary platform, thus allowing the purified nucleic acid to proceed by centrifugal flow from the purification chamber to the one or more reaction chambers; or
in the presence of the one or more elution chambers, indexing of the one or more fluidic cards with the one or more elution chambers being oriented radially outwards and applying rotary movement to the rotary platform, thus allowing the purified nucleic acid to proceed by centrifugal flow from the purification chamber to the one or more elution chambers, and after diluting the purified nucleic acid, indexing of the one or more fluidic cards with the one or more reaction chambers being oriented radially outwards and applying rotary movement to the rotary platform, thus allowing the purified nucleic acid to proceed by centrifugal flow from the one or more elution chambers to the one or more reaction chambers;
(vi) amplifying the nucleic acid in the one or more reaction chambers, particularly by performing a polymerase-chain reaction; and
(vii) indexing of the one or more fluidic cards with the one or more pooling and/or collection chambers being oriented radially outwards and applying rotary movement to the rotary platform, thus allowing the amplified nucleic acid to proceed by centrifugal flow from the one or more reaction chambers to the one or more pooling and/or collection chambers.

10. The method of claim 9, wherein the biological sample is selected from the group consisting of blood, plasma, serum, saliva, urine, nasopharyngeal swab, oropharyngeal swab, and tissue specimens.

11. The method of claim 9 or 10, wherein the purification of the nucleic acid is performed by immobilizing the nucleic acid to a magnetic solid support, particularly to paramagnetic beads.

12. The method of claim 11, further comprising one or more of the following:
(i) releasing of the nucleic acid from the biological sample by providing a reagent for cell lysis to the one or more receiving chambers (x.i) and allowing it to proceed to the purification chamber; and/or
(ii) one or more washing steps of the immobilized nucleic acid by providing a washing reagent to the one or more receiving chambers (x.i) and allowing it to proceed to the purification chamber; and/or
(iii) eluting the immobilized nucleic acid from the magnetic solid support by providing an elution reagent to the one or more receiving chambers (x.i) and allowing it to proceed to the purification chamber.

13. The method of any one of claims 9 to 12, wherein the one or more reaction chambers are provided with reagents for nucleic acid amplification prior to performing step (vi), wherein the one or more reagents for nucleic acid amplification are provided to the one or more receiving chambers (x.v) and allowed to proceed to the one or more reaction chambers via the descending slope from the one or more receiving chambers to the one or more reaction chambers.

14. The method of any one of claims 9 to 13, further comprising: diluting of the amplified nucleic acid by providing a dilution reagent to the one or more receiving chambers (x.v) and allowing it to proceed to the one or more reaction chambers.

15. The method of any one of claims 9 to 14, wherein the combined assay of nucleic acid purification and amplification is performed in a multiplex-format; and/or wherein the amplified nucleic acid represents a nucleic acid library for next generation sequencing.
